Europäisches Patentamt

(19) European Patent Office

Office européen des brevets

(11) Numéro de publication: **0 211 197**
**B1**

(12) **FASCICULE DE BREVET EUROPEEN**

(45) Date de publication du fascicule du brevet: **05.12.90**

(21) Numéro de dépôt: **86108206.3**

(22) Date de dépôt: **16.06.86**

(51) Int. Cl.⁵: **A 61 F 13/15**

(54) **Couche-culotte à jeter munie d'une ceinture élastique réactivée.**

(30) Priorité: **19.06.85 FR 8509344**

(43) Date de publication de la demande:
**25.02.87 Bulletin 87/09**

(45) Mention de la délivrance du brevet:
**05.12.90 Bulletin 90/49**

(84) Etats contractants désignés:
**AT BE CH DE GB IT LI NL**

(56) Documents cités:
**EP-A-0 119 825**
**FR-A-2 087 346**
**FR-A-2 289 131**
**US-A-4 041 949**

(73) Titulaire: **PEAUDOUCE**
**59, Rue de la Vignette**
**F-59126 Linselles (FR)**

(72) Inventeur: **Courtray, Franck**
**110 ter Rue de la Vignette**
**F-59126 Linselles (FR)**

(74) Mandataire: **Casalonga, Axel et al**
**BUREAU D.A. CASALONGA - JOSSE**
**Morassistrasse 8**
**D-8000 München 5 (DE)**

Courier Press, Leamington Spa, England.

## Description

La présente invention a pour objet une couche-culotte à jeter pour enfants en bas âge ainsi qu'éventuellement pour adultes incontinents.

La présente invention a également pour objet certains procédés de fabrication de telles couches-culottes à jeter.

On sait que les couches-culottes qui sont utilisées généralement pour les enfants en bas âge mais aussi pour les adultes incontinents, comprennent sous une forme intégrée, à titre d'éléments principaux, une enveloppe extérieure imperméable mince et souple jouant le rôle de culotte lorsque la couche-culotte est refermée et un matelas absorbant fixé à l'intérieur de l'enveloppe imperméable, recouvert d'un voile interne perméable à l'humidité réalisé par exemple en non-tissé. Des dispositifs d'attaches par adhésif sont en outre prévus sur les bords latéraux de la partie arrière de la couche-culotte afin de refermer cette dernière sur l'enfant au moment de l'utilisation. Une telle couche-culotte que l'on appelle parfois change-complet permet de remplacer à la fois la couche absorbante traditionnelle et la culotte imperméable qui était utilisée en combinaison avec ladite couche. On a déjà prévu également de disposer des éléments élastiques de chaque côté des bords longitudinaux du matelas absorbant ou au voisinage des échancrures permettant le passage des jambes lorsque la couche-culotte est portée. A titre d'exemples de telles couches-culottes munies d'élastiques à l'entrejambe on peut citer le brevet américain No. 3 860 003 et les brests français No. 2 063 794, No. 2 438 434.

Les élastiques utilisés sont posés en continu à l'état tendu sur la majeure partie de leur longueur sur la face interne de la feuille imperméable. Leur fixation se fait en général par collage par exemple au moyen d'un adhésif à haute température qui est rapidement figé lorsque la température s'abaisse de façon à être immédiatement fixé en position tendue sur la majeure partie de sa longueur sur la face interne de la feuille imperméable souple. Ces élastiques sont habituellement utilisés sous la forme de bandelettes minces dont l'une des faces reçoit par intermittence un matériau adhésif à température élevée en fusion. On a également décrit dans le brevet français No. 2 539 274 un procédé permettant la fixation sur une enveloppe mince d'une multitude de brins élastiques individuels en latex naturel.

Ces éléments élastiques sont ensuite découpées en même temps que l'ensemble formé en continu et comprenant la feuille imperméable souple, les différents matelas absorbants et le voile perméable interne. Cette opération de découpe permet la rétraction de la portion des élastiques qui n'est pas collée sur la feuille imperméable de sorte que l'on obtient finalement une couche-culotte dont seules les zones d'entrejambe sont munies d'éléments élastiques, ce qui améliore la tenue de la couche-culotte en respectant parfaitement l'anatomie de l'utilisateur. La couche-culotte reste ainsi parfaitement ajustée à l'enfant malgré les mouvements de ce dernier.

Une difficulté subsiste cependant dans les couches-culottes de ce type couramment employées jusqu'à présent. Malgré l'existence des moyens élastiques d'entrejambe et la présence des attaches adhésives latérales ou constate en effet que la couche-culotte n'est en général pas convenablement ajustée à la ceinture. Lorsque les adhésifs sont posés normalement on constate fréquemment que la couche-culotte baille à la fois sur le devant et sur le derrière de la zone de ceinture dès que l'enfant à bougé pendant un certain temps. De plus, il n'est pas possible d'ajuster correctement les attaches adhésives au moment de la pose.

L'amélioration de l'étanchéité à l'endroit de la ceinture des couches-culottes de ce type peut être obtenue en particulier par repli des bords extrêmes transversaux avant et arrière de la couche-culotte avant utilisation. Des moyens permettant de faciliter ce repli sont décrits par exemple dans les demandes de brevet français No. 84 00 180 et No. 84 00 179. Toutefois ces moyens ne remplissent pas pleinement leur rôle s'il n'est pas possible d'obtenir un ajustement convenable de la couche-culotte à l'endroit de la ceinture.. Des culottes fermées ont été réalisées complètement à l'aide d'un matériau élastique (brevet français No. 2 529 056) ou prévues avec un élément élastique entourant complètement la ceinture (brevet français No. 2 490 079).

La demande de brevet français No. 2 515 004 décrit quant à elle l'utilisation d'un organe d'étanchéité élastique qui joue également un rôle de fermeture grâce à des extensions recouvertes d'adhésif. Ce document ne donne pas d'indication sur la nature de l'élastique utilisable ni surtout sur le mode de pose d'un tel organe élastique dans un procédé en continu.

Le fait de prévoir de manière artisanale un élément élastique au voisinage de la ceinture d'un vêtement fermé tel qu'une culotte est bien connu. Ces articles classiques ne peuvent cependant pas être fabriqués en continu à grande cadence en raison du fait que la pose d'un élément élastique à l'état tendu en position transversale par rapport au défilement longitudinale sur une machine automatique, est extrêmement difficile à concevoir et à réaliser.

Pour résoudre de type de difficulté on a déjà songé (demandes de brevet européen No. 119 827 et No. 119 825) à poser sur le bord extrême transversal des parties arrière et avant d'une couche-culotte, un matériau rétractable à chaud et présentant des caractéristiques d'élasticité à froid, tel que des polyuréthanes ou des compositions à base de mélanges d'élastomères et de résines thermoplastiques dont les utilisations sont décrites à titre général dans les brevet U.S. No. 3 912 565, No. 3 819 401 ou No. 4 303 571. Dans les demandes de brevet européen ci-dessus mentionnées, on pose une bande large transversalement au défilement de la feuille imperméable lors de sa fabrication. Cette bande transversale est réalisée en un matériau élastomère rétractable à chaud comme mentionné précédemment. La pose peut donc se faire dans l'état étiré du matériau, par collage au moyen de lignes longitudinales d'adhésif. On découpe ensuite les couches-culottes, la ligne de découpe passant sensiblement dans l'axe de la bande de matériau élastomère. Il en résulte que la couche-culotte finalement obtenue présente sur sa partie avant et sur sa partie arrière des bords extrêmes munis, après activation du matériau élastomère, d'une pluralité de canaux tubulaires ou fronces ouvertes vers l'extérieur. Des attaches adhésives classiques qui permettent la fermeture de la couche-culotte sont placées quant à elles dans une zone plus éloignée du bord transversal de la partie arrière de la couche-culotte. Il en résulte qu'une traction sur les attaches adhésives ne permet pas d'obtenir un ajustement convenable de la couche-culotte. De plus l'étanchéité au voisinage de la ceinture n'est pas conservée en raison de l'existence des fronces formant des canaux tubulaires qui sont incapables de retenir des fuites éventuelles de liquide.

La présente invention a pour objet de résoudre ces difficultés et de permettre la réalisation d'une couche-culotte qui puisse être convenablement ajustée de manière élastique dans la zone de la ceinture et parfaitement adaptée à la morphologie de l'utilisateur sans que l'étanchéité dans la zone de la ceinture soit en quelque manière que ce soit diminuée.

L'invention a encore pour objet de permettre la réalisation d'une couche-culotte de structure simplifiée permettant une économie sensible de matière tout en facilitant l'ajustement élastique mentionné préalablement.

Enfin l'invention a pour objet un procédé de fabrication de telles couches-culottes permettant la localisation à volonté de zones élastiques et de zones non élastiques dans la partie de la ceinture arrière de la couche-culotte.

Telle qu'elle est revendiquée, la couche-culotte à jeter pour enfants en bas âge et adultes incontinents selon l'invention, comprend une feuille imperméable présentant une partie arrière et une partie avant, un matelas absorbant fixé sur la face interne de la feuille imperméable, au moins un élément élastique de ceinture et deux dispositifs d'attache fixés dans la zone de la partie arrière de la feuille imperméable pour permettre de refermer la couche-culotte au moment de l'utilisation. La couche-culotte comprend aussi une bande mince de ceinture réalisée en un matériau rétractable à chaud et capable après rétraction de présenter des caractéristiques d'élasticité, ladite bande étant disposée transversalement sur la partie arrière de la feuille imperméable en laissant subsister un bord extrême libre de la partie arrière, le bande de ceinture ayant été fixée à la feuille imperméable à l'état non rétracté pendant la fabrication de la couche-culotte par des moyens n'entraînant pratiquement pas de modifications de l'état de ladite bande. Les dispositifs d'attache sont fixés sur les extrémités de ladite bande de ceinture.

Grâce à cette position particulière des dispositifs d'attache et à l'existence d'un bord extrême libre sur la partie arrière de la couche-culotte, bord qui peut par exemple être rabattu afin d'assurer l'étanchéité, on obtient une couche-culotte qui peut être parfaitement ajustée de manière élastique au moment de la fermeture par les dispositifs d'attache. De plus l'utilisation d'un matériau rétractable à chaud permet de faciliter la fabrication.

La fixation de la bande de ceinture sur la face interne de la feuille imperméable est faite de façon que la bande de ceinture se trouve entre la feuille imperméable et la portion arrière du matelas absorbant. De préférence la portion arrière du matelas absorbant n'est alors pas fixée à la feuille imperméable. Dans une variante le bord extrême de la partie arrière du matelas absorbant est toutefois fixé à la feuille imperméable au-delà de la bande de ceinture.

La fixation du matelas absorbant peut être faite par tout moyen, en particulier par collage.

La bande de ceinture peut être fixée à la feuille imperméable par une pluralité de lignes longitudinales d'un matériau adhésif déposé à chaud et tel qu'il conserve ses qualités adhésives après refroidissement.

La bande de ceinture peut également être fixée à la feuille imperméable par soudure réalisée au moyen de l'application d'ultrasons ou par calandrage.

La bande de ceinture, peut avoir une longueur égale à la largeur de la partie arrière de la couche-culotte. Les dispositifs d'attache sont alors fixés sur la zone de bordure latérale de la couche-culotte qui comprend les extrémités de la bande de ceinture.

Dans un mode de réalisation préféré cependant, la bande de ceinture est plus longue et dépasse donc de part et d'autre de la partie arrière de la couche-culotte. Dans ce cas les dispositifs d'attache sont fixés sur les deux extrémités de la bande de ceinture.

La plupart du temps, la couche-culotte présente une forme analogue à un sablier, des échancrures latérales facilitant le passage des jambes. Toutefois, dans un mode de réalisation particulièrement avantageux de l'invention, la feuille imperméable formant l'enveloppe extérieure de la couche-culotte ainsi que le matelas absorbant, présentent une forme générale rectangulaire allongée. Les portions extrêmes de la bande de ceinture dépassent les bords transversaux de la feuille imperméable et les deux dispositifs d'attache sont fixés au bout desdites portions extrêmes de la bande de ceinture. Dans un tel mode de réalisation, la bande de ceinture dépasse donc largement les bords de la couche-culotte et celle-ci n'est donc pas fermée à la ceinture pendant l'utilisation, une échancrure subsistant sur les côtés latéraux pour le passage des jambes et jusque sur les hanches. Il en résulte non seulement une économie de matière mais également une plus grande aisance pour l'utilisateur grâce en particulier à l'ajustement élastique réalisé par la ceinture élastique.

Dans tous les cas, l'activation du matériau de la bande de ceinture par application de chaleur, entraîne comme on l'a dit précédemment, la rétraction du matériau et l'apparition de caractéristiques d'élasticité.

Il est possible, selon une variante de l'invention, d'activer l'intégralité de la bande de ceinture, provoquant ainsi la formation de fronces sur toute la portion de la zone arrière sur laquelle la bande de ceinture est fixée.

Selon une autre variante, on peut au contraire n'activer que certaines portions de la bande de ceinture, en particulier des portions fixées à la partie arrière de la couche-culotte pour former des zones froncées ou des portions de la bande de ceinture dépassant de chaque côté de la couche-culotte pour former des pattes de liaison élastiques pour les dispositifs d'attache.

Les dispositifs d'attache peuvent être de type classique, comportant des éléments adhésifs repliés. On peut également utiliser d'autres types d'attaches tels que dispositifs à accrochage, à pression, etc.

Une voile de non-tissé éventuellement hydrophobe tout en restant perméable à l'humidité, peut avantageusement être fixé par collage sur les zones de la feuille imperméable qui ne sont pas recouvertes par le matelas absorbant et sur la face interne du matelas absorbant.

Des éléments élastiques d'entrejambe peuvent être prévus et sont de préférence disposés longitudinalement de part et d'autre du matelas absorbant au voisinage des bords latéraux dudit matelas.

Les couches-culottes selon l'invention sont de préférence fabriquées par un procédé en continu dans lequel on fait defiler longitudinalement à grande vitesse une bande mince, par exemple en polyéthylène qui joue le rôle d'enveloppe extérieure imperméable pour les couches-culottes. On dépose des lignes continues d'un adhésif à l'état liquide, cet adhésif refroidissant rapidement tout en gardant ses caractéristiques de collage.

On pose des bandes transversales à l'état non étiré sur la bande mince qui défile en continu, le collage de ces bandes transversales étant obtenu par les lignes longitudinales de collage précitées ou par soudage aux ultrasons ou par calandrage. Les bandes transversales ainsi posées ont été obtenues en étirant à chaud un matériau dont les molécules peuvent être orientées uniaxialement, l'ensemble étant fixé dans cette position étirée par application d'un refroidissement rapide.

On pose ensuite éventuellement des bandes ou des fils continus élastiques latéraux enduits d'adhésif puis des matelas absorbants individuels et enfin si on le désire une feuille continue d'un voile non tissé éventuellement hydrophobe mais perméable à l'humidité. On pose enfin des dispositifs d'attache sur les deux extrémités latérales de la bande transversale. On découpe transversalement l'ensemble de façon à laisser subsister un bord libre dans la partie arrière des couches-culottes ainsi formées, entre le bord extrême et la bande transversale de ceinture. On soumet ensuite au moins certaines zones de la bande transversale de ceinture à un traitement thermique suffisant pour provoquer la rétraction de ladite bande à son état d'origine de façon à obtenir des zones activées présentant des caractéristiques élastiques.

La feuille imperméable dont il est question dans la présente invention est une feuille mince souple réalisée dans un matériau imperméable aux liquides. Ce matériau peut, avantageusement, rester toutefois perméable aux gaz et en particulier à l'air. Le voile de non-tissé qui peut être utilisé dans le cadre de l'invention peut, dans certains cas, présenter une certaine élasticite.

L'invention sera mieux comprise à l'étude de la description détaillée de quelques modes de réalisation décrits à titre d'exemples nullement limitatifs et illustrés par les dessins annexés sur lesquels:

la figure 1 est une vue schématique d'une couche-culotte selon l'invention représentée à l'état complètement étiré à la fois dans le sens longitudinale et dans le sens transversal avec des arrachements partiels pour montrer la structure interne;

la figure 2 est une vue en coupe selon II—II de la figure 1 à l'état étiré;

la figure 3 est une vue en coupe analogue à la figure 2 à l'état rétracté;

la figure 4 est une vue en coupe analogue à la figure 3 d'une variante de l'invention;

la figure 5 est une vue schématique montrant la couche-culotte refermée comme pour une utilisation;

la figure 6 est une vue schématique d'une variante d'une couche-culotte représentée comme dans le cas de la figure 1 en position complètement étirée à la fois longitudinalement et transversalement et avec des arrachements partiels pour montrer la structure interne;

la figure 7 est une vue en coupe selon VII—VII de la figure 6 à l'état étiré;

la figure 8 est une vue en coupe analogue à la figure 7 à l'état rétracté;

la figure 9 est une vue montrant la couche-culotte fermée comme pour une utilisation normale;

les figures 10 à 12 montrent schématiquement des variantes d'activation partielle de certaines zones de la bande de ceinture dans une couche-culotte analogue à celle qui est illustrée sur la figure 1;

les figures 13 et 14 sont des vues schématiques analogues montrant l'activation de certaines zones de la bande de ceinture dans une couche-culotte analogue à celle illustrée sur la figure 6.

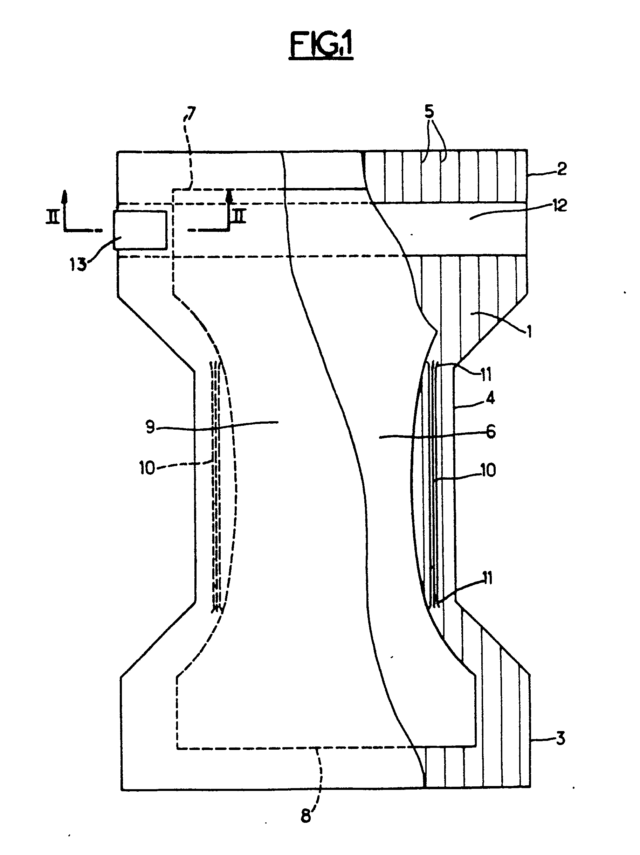

Tel qu'elle est illustrée sur les figures 1 à 3, une couche-culotte comprend une feuille mince imperméable 1 réalisée par exemple en polyéthylène, qui présente une partie arrière 2 et une partie avant 3 séparée par des échancrures latérales 4 permettant le passage des jambes. La forme générale qui en résulte pour la couche-culotte est sensiblement celle d'un sablier. Une pluralité de lignes longitudinales de collage 5 sont appliquées sur la face interne de la feuille 1 au moment de la fabrication sous la forme d'un adhésif liquide qui se fige rapidement tout en gardant ses propriétés adhésives. Un matelas absorbant 6 affectant également une forme générale de sablier et présentant de ce fait une partie arrière 7 et une partie avant 8 est fixé sur la face interne de la feuille imperméable 1 au moyen des lignes longitudinales de collage 5. Cette fixation peut se faire directement ou par l'intermédiaire d'un voile mince en ouate de cellulose de manière classique non représenté sur les figures. Un voile de non-tissé 9 vient recouvrir l'ensemble c'est-à-dire à la fois le matelas absorbant 6 et, dans les zones latérales et extrêmes, la face interne de la feuille imperméable 1. Le voile de non-tissé 9 est fixé par collage sur les portions latérales et extrêmes de la feuille 1 grâce à l'existence des lignes longitudinales 5. Il peut également être fixé à un voile en ouate de cellulose placé de façon usuelle sur la face supérieure du matelas absorbant 6, ce voile étant représenté mais non référencé sur les figures. Le matelas absorbant 6 peut de manière classique être réalisé en une ou plusieurs couches de ouate de cellulose défibrée éventuellement repliées ou posées les unes par rapport aux autres. Il peut avoir également subi un calandrage partiel améliorant la diffusion du liquide et la tenue de la ouage de cellulose. D'autres matériaux absorbants peuvent également être utilisés.

Des éléments élastiques latéraux 10 sont également posés longitudinalement de manière linéaire de chaque côté des bords du matelas absorbant 6 dans la zone de l'échancrure 4. Dans l'exemple illustré sur la figure 1, les éléments élastiques sont constitués par trois brins individuels réalisés par exemple en latex préalablement enduit d'un adhésif liquide à chaud permettant leur fixation par collage en continu sur la face interne de la feuille imperméable 1. Les extrémités 11 des brins élastiques 10 sont libres et se sont rétractées au moment de l'opération finale de découpe transversale de la couche-culotte. Pour obtenir ce résultat l'enduction de l'adhésif sur ces élastiques est faite de manière intermittente selon un procédé classique. On comprendra bien entendu que d'autres types d'éléments élastiques pourraient être utilisés. En particulier, il serait possible d'utiliser un nombre de brins élastiques différent, par exemple 2, 4 brins ou plus. De plus on pourrait remplacer ces brins par d'autres éléments élastiques, par exemple sous forme de bandelettes minces. L'invention ne portant pas sur cette caractéristique de la couche-culotte, on comprendra en outre que les élastiques pourraient également être supprimés.

La couche-culotte se complète par une bande transversale de ceinture 12 qui est disposée dans la partie arrière 2 et collée sur toute la largeur de ladite partie arrière sur la face interne de la feuille imperméable 1. Cette fixation est réalisée dans le mode de réalisation illustré sur la figure 1 par les lignes de collage longitudinales 5. Cette fixation pourrait être réalisées différemment, par exemple par calandrage ou par des points ou de lignes de soudage par ultrasons et d'une manière générale par toute méthode qui permettrait de fixer la bande de ceinture transversale 12 sur toute la largeur de la feuille imperméable 1 sans modifier l'état de la bande transversale 12 au moment de cette opération de fixation.

Deux dispositifs d'attache 13 sont fixés dans la zone de la partie arrière 2 de la couche-culotte à l'endroit de la bande de ceinture transversale 12.

La bande de ceinture 12 est réalisée en un matériau rétractable à chaud et capable après rétraction de présenter des caractéristiques d'élasticité.

Des matériaux de ce type peuvent être obtenus par exemple à base de résine thermoplastique polyuréthane extrudée en couches minces qui sont ensuite étirées à chaud tout en étant maintenues à l'état étiré pendant le refroidissement. L'activation du matériau consiste à soumettre le matériau à un traitement thermique au cours duquel il reprend ses dimensions d'origine tout en conservant en quelque sorte mémorisées ses dimensions à l'état étiré. Dans ces conditions, le matériau présente des caractéristiques d'élasticité. La formation de ce matériau peut être faite par extrusion sous la forme de bande, de couche, de film, de pellicule, etc., suivie ensuite d'un étirage uniaxial entraînant une orientation moléculaire dans le matériau. A titre d'exemple de résine de polyuréthane thermoplastique qui peut être utilisée selon l'invention on peut citer des composés du type "Estane" fabriqué par B. F. Goodrich Co. La température d'orientation moléculaire de ces polyuréthanes varie selon leur structure entre 40 et 145°C. La rétraction ultérieure peut aller jusqu'à 50% des dimensions à l'état étiré. On pourra se reporter pour plus de précision au brevet U.S. No. 3 912 565.

De la même manière, on pourra se reporter au brevet U.S. No. 3 639 917 qui décrit d'autres matériaux rétractables à chaud constitués de blocs polymères élastomères de formule A—B—A où A est un bloc polymère non élastomère et B un bloc polymère élastomère. A est avantageusement un polymère dérivé d'un monomère tel que le styrène, le méthylstyrène ou le chlorostyrène, tandis que le polymère B est avantageusement un polymère du type polyisoprène élastomère.

Les matériaux préférés utilisés selon l'invention sont ceux décrits dans le brevet U.S. No. 4 303 571 auquel on pourra se référer pour plus de précisions. Ces matériaux sont constitués de mélanges de polymères élastomères thermoplastiques filmogènes, et plus particulièrement des mélanges constitués d'élastomères du type copolymère éthylène-propylène ou terpolymère éthylène-propylène-diène avec des résines copolymères thermoplastiques éthylène-acétate de vinyle. Le matériau extrudé sous la forme d'une bande mince est alors soumis comme précédemment à un étirage unidirectionnel à une température d'environ 65°C, la dimension dans le sens de la traction étant augmentée d'environ 400%. Après refroidissement, le polymère se trouve orienté uniaxialement et garde les dimensions étirées qu'il avait acquises à haute température. Il est donc possible de découper ce matériau de façon à réaliser les bandes de ceinture telles que la bande 12 illustrée sur la figure 1 qui peut être posée dans cet état de manière aisée pendant la fabrication en continu des couches-culottes. Un traitement ultérieur de chauffage sur toute la surface du matériau ou sur certaines zones entraîne, pour les zones chauffées à une température de l'ordre de 70°C, un retour du matériau à ses dimensions d'origine avant l'étirage à chaud. Par ailleurs, on constate que la matériau présente des caractéristiques élastiques et peut être étiré d'environ 50%.

On pourra mentionner encore à titre d'exemples de matériaux qu'il est possible d'utiliser dans l'invention ceux décrits dans le brevet U.S. No. 3 819 401 et en particulier des dispersions colloïdales de polychlorure de vinyle dans des plastifiants tels que du diméthylphtalate.

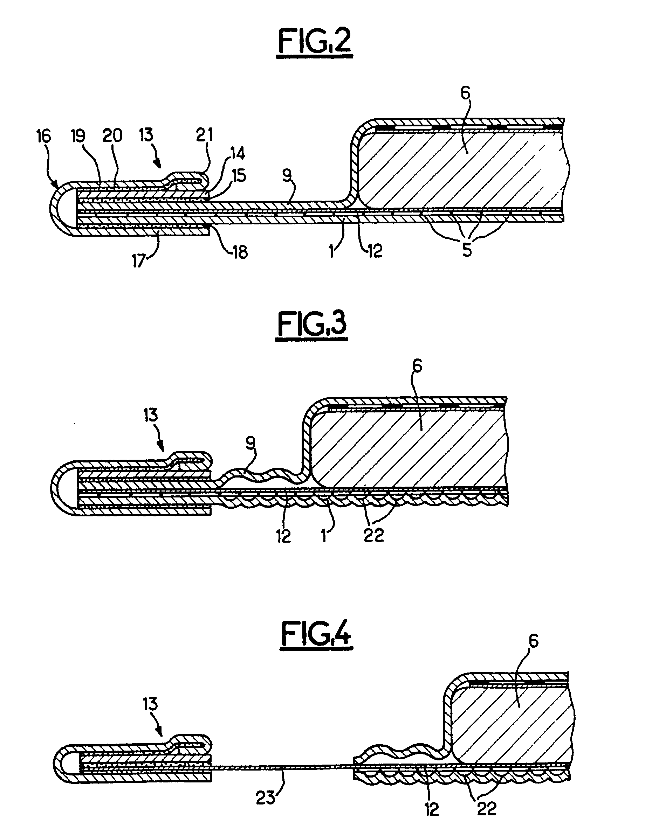

On se reportera maintenant aux figures 2 et 3. Dans l'état étiré illustré sur la figure 2 on voit que le dispositif d'attache 13 est constitué par un élément d'ancrage 14 fixé au moyen d'une couche de colle 15 sur la surface supérieure du bord latéral extrême du voile de non-tissé 9. Un élément de fixation ou de fermeture 16 comprend une portion 17 fixée à demeure sur la face extérieure de la feuille imperméable 1 au moyen d'une couche de colle 18 et un élément de fixation à proprement parlé référence 19 collé de manière détachable par une couche de colle 20 sur la face supérieure de l'élément d'ancrage 14, qui est traitée de façon à réduire l'adhérence. L'élément de fixation 19 est replié sur lui-même à son extrémité 21 de façon à permettre une préhension aisée. Cette structure de type classique est décrite à titre d'exemple on comprendra bien entendu que tout type de dispositif d'attache par adhésif pourrait être utilisé. On pourrait également utiliser d'autres types de dispositif d'attache par exemple le dispositif à accrochage connu sous la marque "Velcro" ou encore des dispositifs à pression ou autres.

La figure 3 montre la déformation de l'ensemble après l'application d'un traitement thermique et rétraction de la bande de ceinture élastique 12. Comme on peut le voir, le rétraction de la bande de ceinture 12 provoque la formation de fronces 22 de la matière de la feuille imperméable 1 qui est fixée sur la bande de ceinture 12 dont les dimensions transversales se sont réduites. On comprendra bien entendu que la figure 3 ne montre pas de manière exacte la réalité, le phénomène étant exagéré pour mieux faire comprendre l'invention. De la même manière des plis se forment dans les portions latérales du voile de non-tissé 9.

Il apparaît dans ces conditions que la couche-culotte finalement obtenue comporte une bande de ceinture élastique sur laquelle sont fixés les dispositifs d'attaches 13. Comme on peut le voir sur la figure 5, la couche-culotte peut alors au moment de l'utilisation être aisément ajustée grâce à une traction convenable sur les attaches 13 qui transmettent l'effort directement à la bande ce ceinture élastique 12 disposée sur la partie arrière de la couche-culotte. Le bord extrême supérieur de la couche-culotte reste pratiquement exempt de tout fronçage, il est donc possible d'assurer une étanchéité convenable au niveau de la ceinture par exemple en rabattant vers l'intérieur ce bord extrême de la partie arrière 2 ainsi que de la partie avant 3.

Dans le mode de réalisation illustré sur les figures 1 à 3, la bande de ceinture 12 a la même largeur que la partie arrière 2 de la couche-culotte et les dispositifs d'attaches sont fixés sur les bords de la couche-culotte à l'endroit de la ceinture élastique 12.

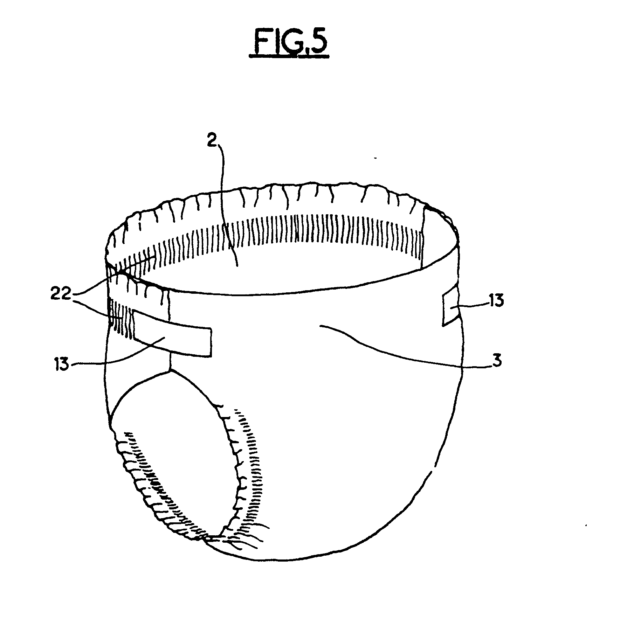

Dans le mode de réalisation illustré sur la figure 4 qui est une coupe analogue à celle de la figure 3, la bande de ceinture 12 présente une longueur plus grande que la largeur totale de la partie arrière de la couche-culotte. Les extrémités latérales 23 de la bande de ceinture élastique 12 dépassent donc de chaque côté de la couche-culotte. Lorsque le matériau de la bande de ceinture élastique 12 a été activé en reprenant ses dimensions initiales tout an aquérant des propriétés élastiques, on obtient donc non seulement la formation de fronces 22 comme précédemment sur la partie arrière de la couche-culotte, mais également une zone élastique 23 constituée par les extrémités de la bande de ceinture 12 se trouvant de chaque côté de la couche-culotte.

Dans ce mode de réalisation, le dispositif d'attache 13 est fixé directement au bout des extrémités 23 de la bande de ceinture 12.

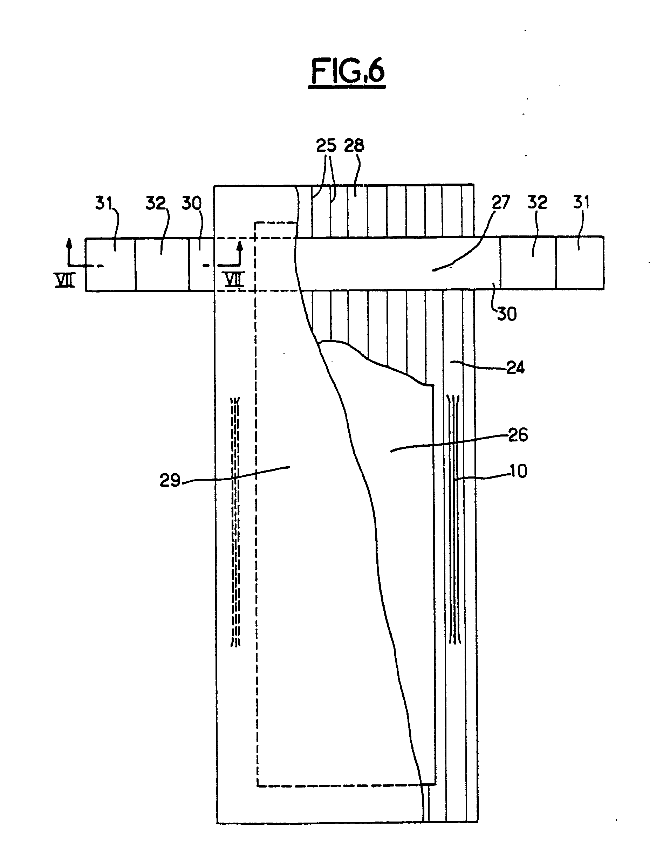

En se rapportant maintenant aux figures 6 à 9, on va décrire un deuxième mode de réalisation de l'invention. Dans ce mode de réalisation, la feuille imperméable 24 est de forme rectangulaire. Sa largeur est inférieure à la largeur totale des parties arrière et avant de la couche-culotte du mode de réalisation précédent. La feuille 24 est également munie de lignes longitudinales de collage 25 qui jouent le même rôle que les lignes 5 des modes de réalisation précédents. Le matelas absorbant 26 est également de forme rectangulaire et il est fixé comme précédemment par les lignes de collage 25 sur la face interne de la feuille imperméable 24. Des éléments élastiques rectilignes 10 sont disposés comme précédemment au voisinage des bords extrêmes latéraux du matelas absorbant 26 sensiblement dans la zone médiane de ce dernier. Comme précédemment, ces éléments élastiques sont collés sur la zone médiane de la feuille 24.

Une bande élastique de ceinture 27 est placée transversalement au voisinage de l'un des petits côtés du rectangle formé par la feuille imperméable 24, en laissant subsister un bord extrême 28 qui présente sensiblement la même largeur dans le sens longitudinal que celle de la bande de ceinture 27. On notera que, comme précédemment, le matelas absorbant 26 vient recouvrir la bande de ceinture 27, son bord extrême étant fixé sur le bord 28 par les lignes de collage 25. On pourrait envisager que le bord extrême du matelas absorbant 26 se trouve sur la bande de ceinture 27.

Un voile de non-tissé 29 vient recouvrir l'ensemble et est collé à la fois sur la face interne de la feuille 24 par les lignes de collage 25 et par l'intermédiaire d'un voile en ouate ce cellulose sur le matelas 26.

La bande de ceinture 27 dépasse largement de chaque côté de la feuille imperméable 24 et comporte donc deux extrémités libres latérales 30. Les bouts de chacune de ces extrémités 30 sont équipés de dispositifs d'attache par adhésif 31.

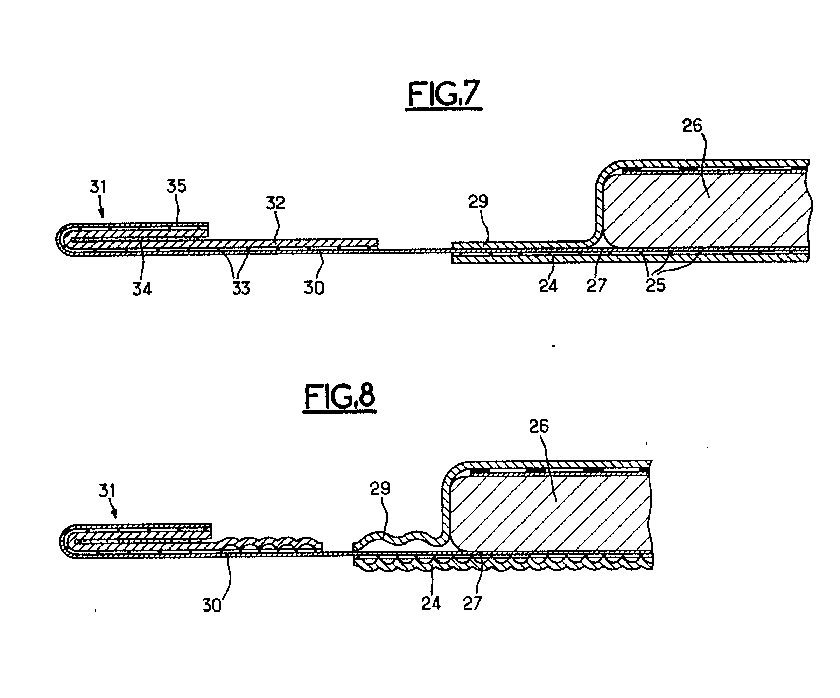

Les vues en coupe des figures 7 et 8 montrent de manière plus précise la structure des extrémités 30 de la bande de ceinture 27. Les extrémités 30 sont revêtues du côté interne par rapport à la couche-culotte d'une pellicule en non-tissé ou en textile 32 qui peut être fixée par des lignes de collage 33 ou des soudures. De cette manière, la surface de contact avec la peau de l'utilisateur est plus douce que dans le cas d'un contact direct avec le matériau de la bande de ceinture élastique 27. Dans l'exemple illustré sur les figures 7 et 8, le dispositif d'attache par adhésif 31 est intégré au bout de l'extrémité 30. Une zone 34 est revêtue d'une couche d'adhésif et le bout de l'extrémité 30 est replié sur l'adhésif 34 de façon à former l'élément de fixation 35 du dispositif d'attache par adhésif.

Là encore, on notera que de nombreux autres types d'attache, que ce soit par adhésif ou par d'autres moyens, pourraient être utilisés.

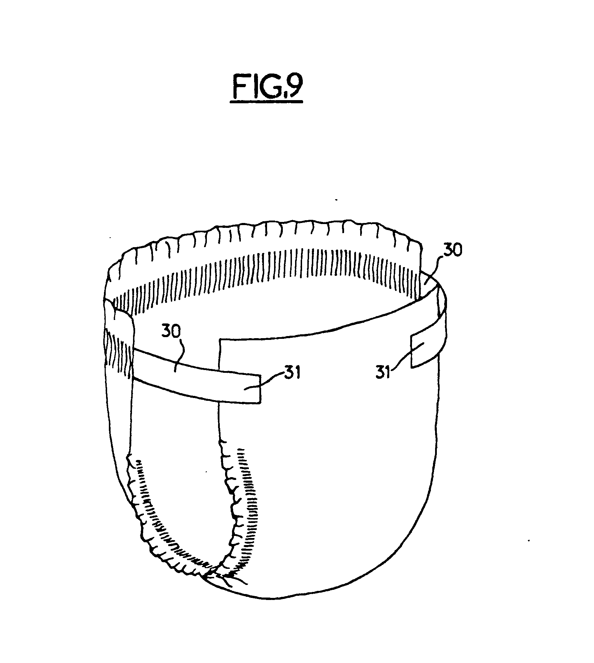

La figure 9 montre schématiquement une telle couche-culotte reformée comme pour une utilisation. On voit que dans ce mode de réalisation, le passage des jambes reste ouvert jusque sur les hanches. Comme précédemment, la zone arrière de la ceinture forme une multitude de fronces élastiques. Les extrémités 30 de la bande de ceinture sont également élastiques et permettent, en combinaison avec les fronces de la partie arrière, d'ajuster parfaitement la couche-culotte au moment de l'utilisation et au moment de la fermeture par les adhésifs 31.

Dans les modes de réalisation précédents, on a supposé que la bande de ceinture était activée sur toute sa surface par un traitement thermique, de sorte qu'elle était entièrement élastique. Il est également possible de n'activer que certaines zones de la bande de ceinture, de façon à obtenir des effets particuliers.

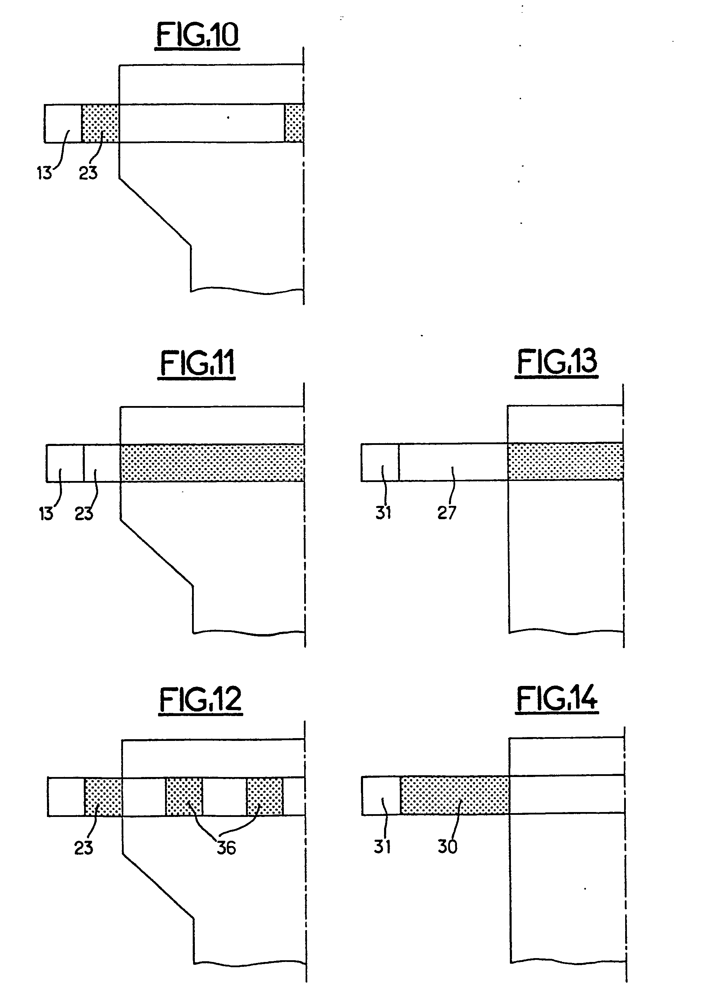

Dans la figure 10 qui illustre une couche-culotte analogue à celle illustrée sur la figure 4, la bande de ceinture n'a été activée que dans sa partie centrale et sur ses extrémités 23. On obtient ainsi une portion froncée dans le creux du dos de l'enfant, qui permet de mieux adapter la couche-culotte à son anatomie. De plus, les extrémités 23 qui portent les dispositifs d'attache 12 permettent d'ajuster parfaitement la couche-culotte au moment de l'utilisation grâce à leur élasticité.

Dans le mode de réalisation illustré sur la figure 11, seule la partie de la bande de ceinture qui est fixée sur la feuille imperméable de la couche-culotte a été activée. Les extrémités 23 non-activées ne sont donc pas élastiques.

Dans le mode de réalisation illustré sur la figure 12, on a procédé à l'activation de certaines zones placées à intervalles réguliers de la bande de ceinture sur la partie arrière de la couche-culotte, ainsi que sur les extrémités 23. Les zones froncées 36 ainsi obtenues permettent, selon leur largeur, de réaliser une élasticité de la partie arrière de la couche-culotte adaptée à chaque cas particulier.

La figure 13 montre le cas d'une couche-culotte ayant la structure de celle illustrée sur la figure 6 dans laquelle seule la partie de la bande de ceinture 27 qui est fixée à la couche-culotte a été activée et forme donc des fronces.

Au contraire, dans le mode de réalisation de la figure 14, seules les parties extrêmes 30 de la bande de ceinture ont été activées et sont donc élastiques. On obtient ainsi un parfait ajustement de la couche-culotte, tandis que la zone arrière en contact avec le dos de l'enfant reste plane.

## Revendications

1. Couche-culotte à jeter pour enfants en bas âge et adultes incontinents, comprenant une feuille imperméable (1) présentant une partie arrière (2) et une partie avant (3), au matelas absorbant (6) fixé sur la face interne de la feuille imperméable, au moins un élément élastique de ceinture et deux dispositifs d'attache (13) fixés dans la zone de la partie arrière de la feuille imperméable pour permettre de refermer la couche-culotte au moment de l'utilisation, la couche-culotte comprenant aussi une bande mince de ceinure (12) réalisée en un matériau rétractable à chaud et capable après rétraction de présenter des caractéristiques d'élasticité, ladite bande étant disposée transverselement sur la partie arrière (2) de la feuille imperméable (1) en laissant subsister un bord extrême libre de la partie arrière, la bande de ceinture ayant été fixée à la feuille imperméable à l'état non rétracté pendant la fabrication de la couche-culotte par des moyens n'entraînement pratiquement pas de modifications de l'état de ladite bande, et que les dispositifs d'attache (13) sont fixés sur les extrémités de ladite bande de ceinture.

2. Couche-culotte selon la revendication 1, caractérisée par le fait que la bande de ceinture est fixée sur toute la largeur de la feuille imperméable par une pluralité de lignes longitudinales d'un matériau adhésif déposé à chaud et conservant ses qualités adhésives après refroidissement, ou par des soudures réalisées au moyen de l'application d'ultrasons ou par calandrage.

3. Couche-culotte selon l'une quelconque des revendications 1 ou 2, caractérisée par le fait que la longueur de la bande de ceinture est égale à la largeur de la partie arrière de la couche-culotte, les dispositifs d'attache étant fixés sur la zone de bordure latérale de la couche-culotte qui comprend les extrémités de la bande de ceinture.

4. Couche-culotte selon la revendication 1 ou 2, caractérisée par le fait que la bande de ceinture présente une longueur supérieure à la largeur de la partie arrière de la couche-culotte et dépasse de part et d'autres de ladite partie arrière, les dispositifs d'attache étant fixés au bout des deux extrémités de la bande de ceinture.

5. Couche-culotte selon la revendication 4, caractérisée par le fait que la feuille imperméable (24) ainsi que le matelas absorbant (26) présentent une forme générale rectangulaire allongée, une échancrure subsistant sur les côtés latéraux pour le passage des jambes et jusque sur les hanches lorsque la couche-culotte est fermée pour son utilisation.

6. Couche-culotte selon l'une quelconque des revendications précédentes, caractérisée par le fait que la totalité de la surface de la bande de ceinture fixée à la feuille imperméable, a été soumise en fin de fabrication de la couche-culotte à un traitement thermique capable d'opérer la rétraction du matériau en conservant à la bande ses qualités d'élasticité.

7. Couche-culotte selon l'une quelconque des revendications 1 à 6, caractérisée par le fait que seules certaines portions de la surface de la bande de ceinture ont été soumises en fin de fabrication de la couche-culotte à un traitement thermique capable d'opérer la rétraction du matériau en conservant à la bande des qualités d'élasticité.

8. Couche-culotte selon la revendication 7, caractérisée par le fait que seules les extrémités de la bande de ceinture dépassant latéralement de la couche-culotte ont été soumises audit traitement thermique.

9. Couche-culotte selon la revendication 7, caractérisée par le fait que seule la portion centrale de la bande de ceinture a été soumis audit traitement thermique.

10. Couche-culotte selon l'une quelconque des revendications précédentes, caractérisée par le fait qu'un voile de non-tissé (9) perméable est fixé par collage sur les zones de la feuille imperméable non recouvertes, par le matelas absorbant.

11. Couche-culotte selon l'une quelconque des revendications précédentes, caractérisée par le fait que des éléments élastiques (10) sont disposés longitudinalement de part et d'autre du matelas absorbant au voisinage de des bords latéraux.

12. Couche-culotte selon l'une quelconque des revendications précédentes, caractérisée par le fait que le matelas absorbant est fixé par collage.

13. Procédé pour la fabrication en continu de couches-culottes selon l'une des revendications précédentes dans lequel on fait défiler longitudinalement à grande vitesse une bande mince jouant le rôle d'enveloppe extérieure imperméable pour les couches-culottes, on dépose des lignes continues d'un adhésif à l'état liquide sur la bande mince, on pose ensuite des matelas absorbants individuels, éventuellement une feuille continue d'un voile non-tissé perméable à l'humidité et enfin des dispositifs d'attache sur les bords de l'ensemble, et dans lequel on pose en outre des bandes transversales à l'état non-étiré sur la bande mince précitée, on fixe lesdites bandes transversales par collage au moyen d'un adhésif à froid ou par soudage aux ultrasons ou par calandrage, les bandes transversales ainsi posées ayant été obtenues en étirant à chaud un matériau dont les molécules peuvent être orientées uniaxialement, l'ensemble étant fixé dans cette position étirée par application d'un refroidissement rapide, et on découpe ensuite transversalement l'ensemble de façon à laisser subsister un bord libre dans la partie arrière des couche-culottes ainsi formées entre le bord extrême et la bande transversal de ceinture, et on soumet finalement au moins certaines zones de la bande transversale de ceinture à un traitement thermique suffisant pour provoquer la rétraction de ladite bande à son état d'origine de façon à obtenir des zones activées présentant des caractéristiques élastiques.

**Patentansprüche**

1. Wegwerfwindelhöschen für Kleinkinder und inkontinente Erwachsene mit einer undurchlässigen Folie (1), die einen hinteren Teil (2) und einen vorderen Teil (3) aufweist, einem saugfähigen Kissen (6), das auf der Innenseite der undurchlässigen Folie befestigt ist, wenigstens einem elastischen Gürtelelement und zwei Verschlüssen (13), die im Bereich des hinteren Teiles der undurchlässigen Folie befestigt sind, damit das Windelhöschen zum Gebrauch geschlossen werden kann, wobei das Windelhöschen weiters ein dünnes Gürtelband (12) besitzt, das aus einem warmschrumpffähigen und nach dem Schrumpfen elastische Eigenschaften aufweisenden Werkstoff hergestellt ist, wobei das Band auf dem hinteren Teil (2) der undurchlässigen Folie (1) quer angeordnet ist und dabei einen freien Außenrand des hinteren Teils frei läßt, wobei das Gürtelband während der Herstellung des Windelhöschens im nicht geschrumpften Zustand an der undurchlässigen Folie durch Mittel befestigt ist, die praktisch keine Änderungen des Zustandes des genannten Bandes bewirken, und daß die Verschlüsse (13) an den Enden des Gürtelbandes befestigt sind.

2. Windelhöschen nach Anspruch 1, dadurch gekennzeichnet, daß das Gürtelband durch mehrere längslaufende Linien aus einem Klebstoff, der warm aufgebracht worden ist und der nach dem Abkühlen seine Klebeigenschaften beibehält, oder durch Schweissungen, die durch Ultraschall oder durch Kalandern erzeugt worden sind, über die ganze Breite der undurchlässigen Folie befestigt ist.

3. Windelhöschen nach einem der Ansprüche 1 oder 2, dadurch gekennzeichnet, daß die Länge des Gürtelbandes gleich der Breite des hinteren Teils des Windelhöschens ist, wobei die Verschlüsse auf dem Bereich des Seitenrandes des Windelhöschens befestigt sind, der die Enden des Gürtelbandes aufweist.

4. Windelhöschen nach Anspruch 1 oder 2, dadurch gekennzeichnet, daß das Gürtelband eine Länge aufweist, die größer ist als die Breite des hinteren Teils des Windelhöschens, und auf beiden Seiten über den hinteren Teil vorsteht, wobei die Verschlüsse am Ende der beiden Endbereiche des Gürtelbandes befestigt sind.

5. Windelhöschen nach Anspruch 4, dadurch gekennzeichnet, daß die undurchlässige Folie (24) und das saugfähige Kissen (26) eine im wesentlichen längliche, rechteckige Form aufweisen, wobei auf den Seiten eine Ausnehmung für den Beindurchtritt verbleibt, die bis zu den Hüften reicht, wenn das Windelhöschen für seine Verwendung geschlossen ist.

6. Windelhöschen nach einem der vorhergehenden Ansprüche, dadurch gekennzeichnet, daß die ganze Fläche des Gürtelbandes, die auf der undurchlässigen Folie befestigt ist, am Ende der Herstellung des Windelhöschens einer Wärmebehandlung unterworfen wurde, um das Schrumpfen des Werkstoffes auszulösen, wobei das Band seine elastischen Eigenschaften beibehält.

7. Windelhöschen nach einem der Ansprüche 1 bis 5, dadurch gekennzeichnet, daß am Ende der Herstellung des Windelhöschens nur bestimmte Abschnitte der Fläche des Gürtelbandes einer Wärmebehandlung unterworfen worden sind, um das Schrumpfen des Werkstoffs auszulösen, wobei das Band seine elastischen Eigenschaften beibehält.

8. Windelhöschen nach Anspruch 7, dadurch gekennzeichnet, daß nur die Enden des Gürtelbandes, die seitliche über das Windelhöschen vorstehen, der genannten Wärmebehandlung unterworfen worden sind.

9. Windelhöschen nach Anspruch 7, dadurch gekennzeichnet, daß nur der mittlere Bereich des Gürtelbandes der genannten Wärmebehandlung unterworfen worden ist.

10. Windelhöschen nach einem der vorhergehenden Ansprüche, dadurch gekennzeichnet, daß ein durchlässiges Vliestuch (9) durch Kleben auf den durch das saugfähige Kissen nicht abgedeckten Bereichen der undurchlässigen Folie befestigt ist.

11. Windelhöschen nach einem der vorhergehenden Ansprüche, dadurch gekennzeichnet, daß die elastischen Elemente (10) in Längsrichtung beiderseits des saugfähigen Kissens in der Nähe seiner Seitenränder angeordnet sind.

12. Windelhöschen nach einem der vorhergehenden Ansprüche, dadurch gekennzeichnet, daß das saugfähige Kissen durch Kleben befestigt ist.

13. Verfahren zum kontinuierlichen Herstellen von Windelhöschen nach einem der vorhergehenden Ansprüche, wobei man mit großer Geschwindigkeit ein dünnes Band in Längsrichtung bewegt, das die undurchlässige Außenhülle für die Windelhöschen bildet, wobei man kontinuierlich Streifen aus einem Klebstoff in flüssigem Zustand auf das dünne Band ablegt, worauf man einzelne saugfähige Kissen, gegebenenfalls eine kontinuierliche Folie eines feuchtigkeitsdurchlässigen Vliesstoffes und schließlich an den Rändern der Anordnung Verschlüsse anbringt, und wobei man überdies nicht gespannte Querbänder auf das oben genannte dünne Band auflegt, wobei man die Querbänder durch Kleben mit Hilfe eines Kaltklebers, durch Ultraschallschweißen oder durch Kalandern befestigt, wobei die so angebrachten Querbänder erhalten worden sind, indem man einen Werkstoff, dessen Moleküle in einer Richtung ausgerichtet werden können, in der Wärme spannt, wobei die Anordnung in dieser gespannten Stellung durch rasches Abkühlen fixiert wird, und wobei man dann die Anordnung quer so durchschneidet, daß man zwischen dem Außenrand und dem quer verlaufenden Gürtelband einen freien Rand im hinteren Teil des so gebildeten Windelhöschens bestehen läßt und wobei man schließlich wenigstens bestimmte Bereiche des quer verlaufenden Gürtelbandes einer Wärmebehandlung unterwirft, die hinreicht, um das Schrumpfen des genannten Bandes in seinen ursprünglichen Zustand zu bewirken, derart, daß aktive Zonen erhalten werden, die elastische Eigenschaften besitzen.

## Claims

1. Disposable napkin for young children and incontinent adults, comprising an impermeable sheet (1) having a rear part (2) and a front part (3), an absorbent padding (6) fixed on the inner face of the impermeable sheet, at least one elastic waistband element and two fastening devices (13) fixed in the zone of the rear part of the impermeable sheet to permit closure of the napkin at the time of use, the napkin also comprising a thin waistband strip (12) made of a heat-retractable material and capable, after retraction, of exhibiting elasticity characteristics, the said strip being arranged transversely on the rear part (2) of the impermeable sheet (1) while leaving an extreme edge of the rear part free, the waistband strip having been fixed to the impermeable sheet in the unretracted state during the manufacture of the napkin by means involving virtually no modifications of the state of the said strip, and in that the fastening devices (13) are fixed on the ends of the said waistband strip.

2. Napkin according to Claim 1, characterized in that the waistband strip is fixed over the entire width of the impermeable sheet by a plurality of longitudinal lines of an adhesive material which is applied while hot and retains its adhesive qualitites after cooling, or by welds produced by means of the application of ultrasound, or by calendering.

3. Napkin according to either one of Claims 1 or 2, characterized in that the length of the waistband strip is equal to the width of the rear part of the napkin, the fastening devices being fixed on the lateral border zone of the napkin which comprises the ends of the waistband strip.

4. Napkin according to Claim 1 or 2, characterized in that the waistband strip has a length greater than the width of the rear part of the napkin and protrudes from both sides of the said rear part, the fastening devices being fixed to the extremity of the two ends of the waistband strip.

5. Napkin according to Claim 4, characterized in that the impermeable sheet (24) and the absorbent padding (26) have an elongated rectangular general shape, a cutout existing on the lateral sides of the legs to pass through up to the hips when the napkin is closed for its use.

6. Napkin according to any one of the preceding claims, characterized in that the entire surface of the waistband strip fixed to the impermeable sheet has been subjected, at the end of manufacture of the napkin, to a heat treatment capable of bringing about the retraction of the material while retaining the elasticity qualities of the strip.

7. Napkin according to any one of Claims 1 to 5, characterized in that only certain portions of the surface of the waistband strip have been subjected, at the end of manufacture of the napkin, to a heat treatment capable of bringing about the retraction of the material while retaining the elasticity qualities of the strip.

8. Napkin according to Claim 7, characterized in that only the ends of the waistband strip protruding laterally from the napkin have been subjected to the said heat treatment.

9. Napkin according to Claim 7, characterized in that only the central portion of the waistband strip has been subjected to the said heat treatment.

10. Napkin according to any one of the preceding claims, characterized in that a permeable nonwoven web (9) is fixed by adhesive bonding on the zones of the impermeable sheet which are not covered by the absorbent padding.

11. Napkin according to any one of the preceding claims, characterized in that elastic elements (10) are arranged longitudinally on both sides of the absorbent padding in the region of its lateral edges.

12. Napkin according to any one of the preceding claims, characterized in that the absorbent padding is fixed by adhesive bonding.

13. Process for the continuous manufacture of napkins according to one of the preceding claims, in which a thin web acting as an impermeable outer covering for the napkins is caused to move past longitudinally at high speed, continuous lines of an adhesive in the liquid state are applied onto the thin web, individual absorbent paddings and optionally a continuous sheet of a nonwoven web permeable to moisture are then laid and finally fastening devices are disposed on the edges of the whole arrangement, and in which transverse strips in the unstretched state are additionally laid on the above-mentioned thin web, the said transverse strips are fixed by adhesive bonding using a cold adhesive or by ultrasonic welding or by calendering, the transverse strips thus laid having been obtained by hot-stretching a material whose molecules may be oriented uniaxially, the whole arrangement being fixed in this stretched position by the application of rapid cooling, and the whole arrangement in then cut transversely so as to leave a free edge in the rear part of the napkins thus formed, between the extreme edge and the transverse waistband strip, and at least certain zones of the transverse waistband strip are finally subjected to a heat treatment which is sufficient to cause the retraction of the said strip to its original state so as to obtain activated zones exhibiting elastic characteristics.

# FIG.1

# FIG.2

# FIG.3

# FIG.4

## FIG.5

# FIG.6

FIG.7

FIG.8

# FIG.9

## FIG.10

13  23

## FIG.11

13  23

## FIG.13

31  27

## FIG.12

23  36

## FIG.14

31  30